# EUROPÄISCHE PATENTSCHRIFT

(11) **EP 0 865 436 B1**
(45) Veröffentlichungstag und Bekanntmachung des Hinweises auf die Patenterteilung: **03.05.2000**
(21) Anmeldenummer: 96941017.4
(22) Anmeldetag: 25.11.1996
(51) Int. Cl.: C07D 285/01, C07D 417/12

(54) **DITHIAZOLDIOXIDE UND IHRE VERWENDUNG ALS MIKROBIZIDE**
DITHIAZOLDIOXIDES AND THE USE THEREOF AS MICROBICIDES
DIOXYDES DE DITHIAZOLE ET LEUR UTILISATION EN TANT QUE MICROBICIDES

(30) Priorität: 07.12.1995 DE 19545635
(43) Veröffentlichungstag der Anmeldung: 23.09.1998
(73) Patentinhaber: BAYER AG, 51368 Leverkusen (DE)
(72) Erfinder: UHR, Hermann, D-47800 Krefeld (DE); STENZEL, Klaus, D-40595 Düsseldorf (DE); KUGLER, Martin, D-42799 Leichlingen (DE); SCHRAGE, Heinrich, D-47829 Krefeld (DE)
(86) Internationale Anmeldenummer: EP9605187
(87) Internationale Veröffentlichungsnummer: WO9720830

(56) Entgegenhaltungen:
- DE-A- 2 608 488
- US-A- 3 345 374
- US-A- 4 271 306
- US-A- 4 962 102

## Beschreibung

Die Erfindung betrifft neue Dithiazoldioxide, Verfahren zu ihrer Herstellung und die Verwendung im Pflanzen- und Materialschutz.

Dithiazole werden bereits beschrieben, eine biologische Wirkung ist nicht erwähnt. 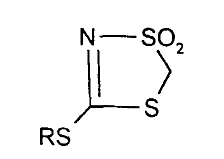 (s. K. Dickoré, Lieb. Ann. Chem. 671, 135 (1964); US-PA 3 345 374).

Überraschenderweise wurde nun gefunden, daß die neuen Verbindungen der allgemeinen Formel (I) 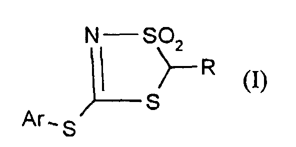 in welcher
- R: für Wasserstoff oder gegebenenfalls substituiertes Alkyl, Alkenyl oder Alkinyl steht und
- Ar: für gegebenenfalls substituiertes Aryl steht,
hervorragend zum Schutz von Pflanzen und Materialien geeignet sind.

Die erfindungsgemäßen Verbindungen sind durch die Formel (I) allgemein definiert. Bevorzugt sind Verbindungen der Formel (I), in welcher
- R: für Wasserstoff, geradkettiges und verzweigtes Alkyl mit 1 ist 10 Kohlenstoffatomen, geradkettiges oder verzweigtes Alkenyl mit 2 bis 10 Kohlenstoffatomen oder geradkettiges oder verzweigtes Alkinyl mit 2 bis 10 Kohlenstoffatomen, welches gegebenenfalls ein- bis mehrfach, gleich oder verschieden substituiert ist durch Halogen, Alkoxy mit 1 bis 6 Kohlenstoffatomen, Halogenalkoxy mit 1 bis 6 Kohlenstoffatomen und 1 bis 9 gleichen oder verschiedenen Halogenatomen, Alkylthio mit 1 bis 6 Kohlenstoffatomen, Halogenalkylthio mit 1 bis 6 Kohlenstoffatomen und 1 bis 9 gleichen oder verschiedenen Halogenatomen, Acyl mit 1 bis 6 Kohlenstoffatomen, Acyloxy mit 1 bis 6 Kohlenstoffatomen, (Alkoxy)carbonyl mit 1 bis 6 Kohlenstoffatomen, Amino, welches gegebenenfalls gleich oder verschieden substituiert ist durch Alkyl oder Aryl, gegebenenfalls substituiertes Phenoxy, Aryl, Pyridyl oder Pyridyloxy, Nitro oder Cyano, steht
und
- Ar: für Aryl steht,
welches gegebenenfalls ein- bis fünffach substituiert ist durch Halogen, Alkyl mit 1 bis 10 Kohlenstoffatomen, Halogenalkyl mit 1 bis 8 Kohlenstoffatomen und 1 bis 8 gleichen oder verschiedenen Halogenatomen, Alkoxy mit 1 bis 10 Kohlenstoffatomen, Halogenalkoxy mit 1 bis 8 Kohlenstoffatomen und 1 bis 8 gleichen oder verschiedenen Halogenatomen, Alkylthio mit 1 bis 10 Kohlenstoffatomen, Halogenalkylthio mit 1 bis 8 Kohlenstoffatomen und 1 bis 8 gleichen oder verschiedenen Halogenatomen, Amino, Monoalkylamino mit geradkettigen oder verzweigten Alkylresten mit 1 bis 6 Kohlenstoffatomen, Dialkylamino mit gleichen oder verschiedenen, geradkettigen oder verzweigten Alkylresten mit je 1 bis 6 Kohlenstoffatomen, Cycloalkyl mit 1 bis 6 Kohlenstoffatomen, Methylendioxy, Difluormethylendioxy, Chlorfluormethylendioxy, Dichlormethylendioxy, Nitro oder Cyano.

Besonders bevorzugt sind Verbindungen der Formel (I), in welcher
- R: für Wasserstoff, geradkettiges oder verzweigtes Alkyl mit 1 bis 8 Kohlenstoffatomen, geradkettiges oder verzweigtes Alkenyl mit 2 bis 8 Kohlenstoffatomen oder geradkettiges oder verzweigtes Alkinyl mit 2 bis 8 Kohlenstoffatomen, welches gegebenenfalls ein- bis vierfach, gleich oder verschieden substituiert ist durch Fluor, Chlor, Alkoxy mit 1 bis 5 Kohlenstoffatomen, Halogenalkoxy mit 1 bis 5 Kohlenstoffatomen und 1 bis 5 Fluor- und/oder Chloratomen, Alkylthio mit 1 bis 5 Kohlenstoffatomen, Halogenalkylthio mit 1 bis 5 Kohlenstoffatomen und 1 bis 5 Fluor- und/oder Chloratomen, Acyl mit 1 bis 5 Kohlenstoffatomen, Acyloxy mit 1 bis 5 Kohlenstoffatomen, Alkoxycarbonyl mit 1 bis 5 Kohlenstoffatomen, Amino, welches gegebenenfalls gleich oder verschieden substituiert ist durch Alkyl mit 1 bis 4 Kohlenstoffatomen und/oder Phenyl, gegebenenfalls substituiertes Phenoxy, Aryl, Pyridyl, Pyridyloxy, Nitro oder Cyano, steht
und
- Ar: für Phenyl steht, welches gegebenenfalls ein- bis vierfach substituiert ist durch Fluor, Chlor, Alkyl mit 1 bis 8 Kohlenstoffatomen, Halogenalkyl mit 1 bis 6 Kohlenstoffatomen und 1 bis 6 Fluor- und/oder Chloratomen, Alkoxy mit 1 bis 8 Kohlenstoffatomen, Halogenalkoxy mit 1 bis 6 Kohlenstoffatomen und 1 bis 6 Fluor- und/oder Chloratomen, Alkylthio mit 1 bis 8 Kohlenstoffatomen, Halogenalkylthio mit 1 bis 6 Kohlenstoffatomen und 1 bis 6 Fluor- und/oder Chloratomen, Amino, Monoalkylamino mit Alkylresten von 1 bis 4 Kohlenstoffatomen, Dialkylamino mit gleichen oder verschiedenen Alkylresten mit jeweils 1 bis 4 Kohlenstoffatomen, Cycloalkyl mit 1 bis 6 Kohlenstoffatomen, Methylendioxy, Difluormethylendioxy, Chlorfluormethylendioxy, Dichlormethylendioxy, Nitro oder Cyano.

Ganz besonders bevorzugt steht R für Wasserstoff, Methyl, Ethyl, n- und i-Propyl, n-, s-, i- und t-Butyl, Allyl und Propargyl, welche gegebenenfalls substituiert sind durch Fluor und/oder Chlor, Methoxy oder Methylthio, und Ar für Phenyl steht, welches gegebenenfalls ein- bis dreifach durch Chlor, Fluor, Methyl, Ethyl, n- und i-Propyl, n-, s-, i- und t-Butyl, Methoxy, Ethoxy, Trifluormethyl, Trifluormethoxy, Cyano und/oder Phenoxy substituiert ist.

Es wurde außerdem gefunden, daß man die Verbindungen der Formel (I) erhält, wenn man die Salze der allgemeinen Formel (II) 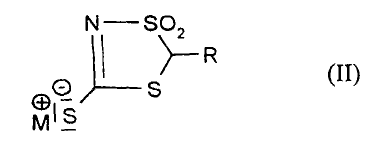 in welcher
- R: die oben angegebene Bedeutung hat und
- M^{⊕}: für ein Alkali- oder Erdalkaliion, im besonderen Na⁺, K⁺ steht,
mit Diazoniumsalzen der allgemeinen Formel (III)

Ar - N ≡ N^{⊕} A^{⊖} (III)

in welcher
- Ar: die oben angegebene Bedeutung hat und
- A^{⊖}: für das Anion einer Mineralsäure steht, in wäßrig/alkalischer Lösung, gegebenenfalls in Gegenwart eines Katalysators umsetzt.

Vorzugsweise gibt man zu einer Lösung von (II) eine Base und gegebenenfalls einen Katalysator und dann die Diazoniumsalzlösung (III). Als Basen werden vorzugsweise Alkalihydroxide wie beispielsweise Kaliumhydroxid oder Natriumhydroxid eingesetzt. Als Katalysatoren kann man alle Katalysatoren einsetzen, die den Austausch der Diazoniumfunktion gegen schwefelhaltige Reste fördert.

Vorzugsweise werden Cu(I)-Salze oder Kupferpulver eingesetzt. Die Temperatur während der Zugabe der Diazoniumsalzlösung kann über einen breiten Bereich variiert werden. Im allgemeinen arbeitet man zwischen -30°C und +60°C, vorzugsweise zwischen -20°C und +40°C. Die Herstellung der Diazoniumsalzlösung aus Anilinen erfolgt nach Literaturmethoden.

Die Salze der allgemeinen Formel (II) lassen sich ebenfalls nach literaturbekannten Methoden herstellen (s. hierzu Literaturstellen S. 1). Man kann entweder in fester Form isolierte Salze der Formel (II) einsetzen oder insitu hergestellte Lösungen.

Die erfindungsgemäßen Wirkstoffe weisen eine starke mikrobizide Wirkung auf und können zur Bekämpfung unerwünschter Mikroorganismen, wie Fungi und Bakterien im Pflanzenschutz und im Materialschutz eingesetzt werden.

Unter technischen Materialien sind im vorliegenden Zusammenhang nicht-lebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe bzw. Mittel gegen Bakterien, Pilze, insbesondere Schimmelpilze, sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puteana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriff fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaaerotheca-Arten, wie beispielsweise Sphaerotheca fuliginea;
Podosphaera-Arten, wie beispielsweise podosphaera leucotricha;
Venturia-Arten, wie beispielsweise Venturia inaequalis;
Pyrenophora-Arten, wie beispielsweise Pyrenophora teres oder Pyrenophora graminea (Konidienform: Drechslera, Synonym: Helminthosporium);
Cochliobolus-Arten, wie beispielsweise Cochliobolus sativus (Konidienform: Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-arten, wie beispielsweise Ustilago nuda oder Ustilago avenae;
Pellicularia-Arten, wie beispielsweise Pellicularia sasakii;
Pyyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe erlaubt auch eine Behandlung von Pflanzen in den zur Bekämpfung von Pflanzenkrankheiten notwendigen Konzentrationen, wobei eine Behandlung von oberirdischen Pflanzenteilen, sowie auch eine Behandlung von Pflanz- und Saatgut und des Bodens durchgeführt werden kann.

Die Wirkstoffe der Formel (I) können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole und Feinstverkapselungen in polymeren Stoffen.

Diese Formulierungen bzw. Mittel werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Wirksamkeit und das Wirkungsspektrum der Wirkstoffe der Formel (I) bzw. die daraus herstellbaren Mittel, Vorprodukte oder ganz allgemein Formulierungen kann erhöht werden, wenn gegebenenfalls weitere antimikrobiell wirksame Verbindungen, Fungizide, Bakterizide, Herbizide, Insektizide oder andere Wirkstoffe zur Vergrößerung des Wirkungsspektrums oder Erzielung besonderer Effekte wie z.B. des zusätzlichen Schutzes vor Insekten zugesetzt werden. Diese Mischungen können ein breiteres Wirkungsspektrum besitzen als die erfindungsgemäßen Verbindungen.

In vielen Fällen erhält man dabei synergistische Effekte, d.h. die Wirksamkeit der Mischung ist größer als die Wirksamkeit der Einzelkomponenten. Besonders günstige Mischungspartner sind z.B. die folgenden Verbindungen:

### Triazole wie:

Amitrole, Azocyclotin, BAS 480F, Bitertanol, Difenoconazole, Fenbuconazole, Fenchlorazole, Fenethanil, Fluquinconazole, Flusilazole, Flutriafol, Imibenconazole, Isozofos, Myclobutanil, Metconazole, Epoxyconazole, Paclobutrazol, Penconazole, Propioconazole, (±)-cis-1-(4-chlorphenyl)-2-(1H-1,2,4-triazol-1-yl)-cycloheptanol, Tetraconazole, Triadimefon, Triadimenol, Triapenthenol, Triflumizole, Triticonazole, Uniconazole sowie deren Metallsalze und Säureaddukte.

### Imidazole wie:

Imazalil, Pefurazoate, Prochloraz, Triflumizole, 2-(1-tert-Butyl)-1-(2-chlorphenyl)-3-(1,2,4-triazol-1-yl)-propan-2-ol, Thiazolcarboxanilide wie 2',6'-Dibromo-2-methyl-4-trifluoromethoxy-4'-trifluoromethyl-1,3-thiazole-5-carboxanilide, 1-Imidazolyl-1-(4'-chlorophenoxy)-3,3-dimethylbutan-2-on sowie deren Metallsalze und Säureaddukte.

Methyl(E)-2-[2-[6-(2-cyanophenoxy)pyrimidin-4-yloxy]phenyl]3-methoxyacrylate, methyl(E)-2-[2-[6-(2-thioamidophenoxy)pyrimidin-4-yloxy]phenyl]-3-methoxyacrylate, methyl(E)-2-[2-[6-(2-fluorophenoxy)pyrimidin-4-yloxy]phenyl]-3-methoxyacrylate, methyl(E)-2-[2-[6-(2,6-difluorophenoxy)pyrimidin-4-yloxy]phenyl]-3-methoxyacrylate, methyl(E)-2-[2-[3-(pyrimidin-2-yloxy)phenoxy]phenyl]-3-methoxyacrylate, methyl(E)-2-[2-[3-(5-methylpyrimidin-2-yloxy)-phenoxy]phenyl]-3-methoxyacrylate, methyl(E)-2-[2-[3-(phenyl-sulfonyloxy)phenoxy]phenyl]-3-methoxyacrylate, methyl(E)-2-[2-[3-(4-nitrophenoxy)phenoxy]phenyl]-3-methoxyacrylate, methyl(E)-2-[2-phenoxyphenyl]-3-methoxyacrylate, methyl(E)-2-[2-(3,5-dimethylbenzoyl)pyrrol-1-yl]-3-methoxyacrylate, methyl(E)-2-[2-(3-methoxyphenoxy)phenyl]-.3-methoxyacrylate, methyl(E)-2-[2-(2-phenylethen-1-yl)-phenyl]-3-methoxyacrylate, methyl(E)-2-[2-(3,5-dichlorophenoxy)pyridin-3-yl]-3-methoxyacrylate, methyl(E)-2-(2-(3-(1,1 2,2-tetrafluoroethoxy)phenoxy)phenyl)-3-methoxyacrylate, methyl(E)-2-(2-[3-(alpha-hydroxybenzyl)phenoxy]phenyl)-3-methoxyacrylate, methyl(E)-2-(2-(4-phenoxypyridin-2-yloxy)phenyl)-3-methoxyacrylate, methyl(E)-2-[2-(3-n-propyloxyphenoxy)phenyl]3-methoxyacrylate, methyl(E)-2-[2-(3-isopropyloxyphenoxy)phenyl]-3-methoxyacrylate, methyl(E)-2-[2-[3-(2-fluorophenoxy)phenoxy]phenyl]-3-methoxyacrylate, methyl(E)-2-[2-(3-ethoxyphenoxy)phenyl]-3-methoxyacrylate, methyl(E)-2-[2-(4-tert.-butylpyridin-2-yloxy)phenyl]-3-methoxyacrylate, methyl(E)-2-[2-[3-(3-cyanophenoxy)phenoxy]phenyl]-3-methoxyacrylate, methyl(E)-2-[2-(3-methylpyridin-2-yloxymethyl)phenyl]-3-methoxyacrylate, methyl(E)-2-[2-[6-(2-methylphenoxy)pyrimidin-4-yloxy]phenyl]-3-methoxyacrylate, methyl(E)-2-[2-(5-bromopyridin-2-yloxymethyl)phenyl]-3-methoxyacrylate, methyl(E)-2-[2-(3-(3-iodopyridin-2-yloxy)phenoxy)phenyl]-3-methoxyacrylate, methyl(E)-2-[2-[6-(2-chloropyridin-3-yloxy)pyrimidin-4-yloxy]phenyl]-3-methoxyacrylate, (E),(E)methyl-2-[2-(5,6-dimethylpyrazin-2-ylmethyloximinomethyl)phenyl]-3-methoxyacrylate, (E)-methyl-2-{2-[6-(6-methylpyridin-2-yloxy)pyrimidin-4-yloxy]phenyl}-3-methoxyacrylate, (E),(E)methyl-2-{2-(3-methoxyphenyl)methyloximinomethyl]phenyl}-3-methoxyacrylate, (E)methyl-2-{2-(6-(2-azidophenoxy)-pyrimidin-4-yloxy]phenyl}3-methoxyacrylate, (E),(E)methyl-2-{2-[6-phenylpyrimidin-4-yl)-methyloximinomethyl]phenyl}-3-methoxyacrylate, (E),(E)methyl-2-{2-[(4-chlorophenyl)-methyloximinomethyl]phenyl}-3-methoxyacrylate, (E)methyl-2-{2-[6-(2-n-propylphenoxy)-1,3,5-triazin-4-yloxy]phenyl}-3-methoxyacrylate, (E),(E)methyl-2-{2-[(3-nitrophenyl)methyloximinomethyl]phenyl}-3-methoxyacrylate;

### Succinat-Dehydrogenase Inhibitoren wie:

Fenfuram, Furcarbanil, Cyclafluramid, Furmecyclox, Seedvax, Metsulfovax, Pyrocarbolid, Oxycarboxin, Shirlan, Mebenil (Mepronil), Benodanil, Flutolanil (Moncut);
Naphthalin-Derivate wie Terbinafine, Naftifine, Butenafine, 3-Chloro-7-(2-aza-2,7,7-trimethyl-oct-3-en-5-in);
Sulfenamide wie Dichlofluanid, Tolylfluanid, Folpet, Fluorfolpet; Captan, Captofol;
Benzimidazole wie Carbendazim, Benomyl, Furathiocarb, Fuberidazole, Thiophonatmethyl, Thiabendazole oder deren Salze;
Morpholinderivate wie Tridemorph, Fenpropimorph, Falimorph, Dimethomorph, Dodemorph, Aldimorph, Fenpropidin und ihre arylsulfonsauren Salze, wie z.B. p-Toluolsulfonsäure und p-Dodecylphenyl-sulfonsäure;
Dithiocarbamate, Cufraneb, Ferbam, Mancopper, Mancozeb, Maneb, Metam, Metiram, Thiram Zeneb, Ziram;
Benzthiazole wie 2-Mercaptobenzothiazol;
Benzamide wie 2,6-Dichloro-N-(4-trifluoromethylbenzyl)-benzamide;
Borverbindungen wie Borsäure, Borsäureester, Borax;
Formaldehyd und Formaldehydabspaltende Verbindungen wie Benzylalkoholmono(poly)-hemiformal, Oxazolidine, Hexa-hydro-S-triazine, N-Methylolchloracetamid, Paraformadehyd, Nitropyrin, Oxolinsäure, Tecloftalam;
Tris-N-(cyclohexyldiazeniumdioxy)-aluminium, N-(Cyclo-hexyldiazeniumdioxy)-tributylzinn bzw. K-Salze, Bis-N-(cyclohexyldiazeniumdioxy)-kupfer;
N-Methylisothiazolin-3-on, 5-Chlor-N-methylisothiazolin-3-on, 4,5-Dichloro-N-octylisothiazolin-3-on, N-Octyl-isothiazolin-3-on, 4,5-Trimethylen-isothiazolinone, 4,5-Benzisothiazolinone, N-Methylolchloracetamid;
Aldehyde wie Zimtaldehyd, Formaldehyd, Glutardialdehyd, β-Bromzimtaldehyd;
Thiocyanate wie Thiocyanatomethylthiobenzothiazol, Methylenbisthiocyanat, usw;
quartäre Ammoniumverbindungen wie Benzyldimethyltetradecylammoniumchlorid, Benzyldimethyldodecylammoniumchlorid, Didecyldimethaylammoniumchlorid;
Iodderivate wie Diiodmethyl-p-tolylsulfon, 3-Iod-2-propinyl-alkohol, 4-Chlorphenyl-3-iodpropargylformal, 3-Brom-2,3-diiod-2-propenylethylcarbamat, 2,3,3-Triiodallylalkohol, 3-Brom-2,3-diiod-2-propenylalkohol, 3-Iod-2-propinyl-n-butylcarbamat, 3-Iod-2-propinyl-n-hexylcarbamat, 3-Iod-2-propinyl-cyclohexylcarbamat, 3-Iod-2-propinyl-phenylcarbamat;
Phenolderivate wie Tribromphenol, Tetrachlorphenol, 3-Methyl-4-chlorphenol, 3,5-Dimethyl-4-chlorphenol, Phenoxyethanol, Dichlorphen, o-Phenylphenol, m-Phenylphenol, p-Phenylphenol, 2-Benzyl-4-chlorphenol und deren Alkali- und Erdalkalimetallsalze;
Mikrobizide mit aktivierter Halogengruppe wie Chloracetamid, Bronopol, Bronidox, Tectamer wie 2-Brom-2-nitro-1,3-propandiol, 2-Brom-4'-hydroxy-acetophenon, 2,2-Dibrom-3-nitril-propionamid, 1,2-Dibrom-2,4-dicyanobutan, β-Brom-β-nitrostyrol;
Pyridine wie 1-Hydroxy-2-pyridinthion (und ihre Na-, Fe-, Mn-, Zn-Salze), Tetrachlor-4-methylsulfonylpyridin, Pyrimethanol, Mepanipyrim, Dipyrithion, 1-Hydroxy-4-methyl-6-(2,4,4-trimethylpentyl)-2(1H)-pyridin;
Metallseifen wie Zinn-, Kupfer-, Zinknaphtenat, -octoat, 2-ethylhexanoat, -oleat, -phosphat, -benzoat;
Metallsalze wie Kupferhydroxycarbonat, Natriumdichromat, Kaliumdichromat, Kaliumchromat, Kupfersulfat, Kupferchlorid, Kupferborat, Zinkfluorosilikat, Kupferfluorosilikat;
Oxide wie Tributylzinnoxid, Cu₂O, CuO, ZnO;
Dialkyldithiocarbamate wie Na- und Zn-Salze von Dialkyldithiocarbamaten, Tetramethylthiuramdisulfid, Kalium-N-methyl-dithiocarbamat;
Nitrile wie 2,4,5,6-Tetrachlorisophthalodinitril, Dinatrium-cyano-dithioimidocarbamat;
Chinoline wie 8-Hydroxychinolin und deren Cu-Salze;
Mucochlorsäure, 5-Hydroxy-2(5H)-furanon;
4,5-Dichlorodithiazolinon, 4,5-Benzdithiazolinon, 4,5-Trimethylendithiazolinon, 4,5-Dichlor-(3H)-1,2-dithiol-3-on, 3,5-Dimethyl-tetrahydro-1,3,5-thiadiazin-2-thion, N-(2-p-Chlorbenzoylethyl)-hexaminiumchlorid, Kalium-N-hydroxymethyl-N'-methyl-dithiocarbamat,
2-Oxo-2-(4-hydroxy-phenyl)acethydroximsäure-chlorid,
Phenyl-(2-chlor-cyan-vinyl)sulfon,
Phenyl-(1,2-dichlor-2-cyan-vinyl)sulfon;
Ag, Zn oder Cu-haltige Zeolithe allein oder eingeschlossen in polymere Wirkstoffe.

Ganz besonders bevorzugt sind Mischungen mit
Azaconazole, Bromuconazole, Cyproconazole, Dichlobutrazol, Diniconazole, Hexaconazole, Metaconazole, Penconazole, Propiconazole, Tebuconazole, Methyl(E)-methoximino[α-(o-tolyloxy)-o-tolyl)]acetate, Methyl-(E)-2-{2-[6-(2-cyanphenoxy)-pyrimidin-4-yl-oxy]phenyl}-3-methoxyacrylat, Methfuroxam, Carboxin, Fenpiclonil, 4-(2,2-Difluoro-1,3-benzodioxol-4-yl)-1H-pyrrol-3-carbonitril, Butenafine, Imazalil, N-Methyl-isothiazolin-3-on, 5-Chlor-N-methylisothiazolin-3-on, N-Octylisothiazolin-3-on, Benzisothiazolinone, N-(2-Hydroxypropyl)-amino-methanol, Benzylalkohol-(hemi)-formal, Glutaraldehyd, Omadine, Dimethyldicarbonat, und/oder 3-Iodo-2-propinyl-n-butylcarbamate.

Desweiteren werden auch gut wirksame Mischungen mit den folgenden Wirkstoffen hergestellt:

### Fungizide:

Acypetacs, 2-Aminobutane, Ampropylfos, Anilazine, Benalaxyl, Bupirimate, Chinomethionat, Chloroneb, Chlozolinate, Cymoxanil, Dazomet, Diclomezine, Dichloram, Diethofencarb, Dimethirimol, Diocab, Dithianon, Dodine, Drazoxolon, Edifenphos, Ethirimol, Etridiazole, Fenarimol, Fenitropan, Fentin acetate, Fentin Hydroxide, Ferimzone, Fluazinam, Fluromide, Flusulfamide, Flutriafol, Fosetyl, Fthalide, Furalaxyl, Guazatine, Hymexazol, Iprobenfos, Iprodione, Isoprothiolane, Metalaxyl, Methasulfocarb, Nitrothal-isopropyl, Nuarimol, Ofurace, Oxadiyl, Perflurazoate, Pencycuron, Phosdiphen, Pimaricin, Piperalin, Procymidone, Propamocarb, Propineb, Pyrazophos, Pyrifenox, Pyroquilon, Quintozene, Tar Oils, Tecnazene, Thicyofen, Thiophanate-methyl, Tolclofos-methyl, Triazoxide, Trichlamide, Tricyclazole, Triforine, Vinclozolin.

### Insektizide:

Phosphorsäureester wie Azinphos-ethyl, Azinphos-methyl, α-1(4-Chlorphenyl)-4-(O-ethyl, S-propyl)phosphoryloxy-pyrazol, Chlorpyrifos, Coumaphos, Demeton, Demeton-S-methyl, Diazinon, Dichlorvos, Dimethoate, Ethoate, Ethoprophos, Etrimfos, Fenitrothion, Fenthion, Heptenophas, Parathion, Parathion-methyl, Phosalone, Phoxim, Pirimiphos-ethyl, Pirimiphos-methyl, Profenofos, Prothiofos, Sulfprofos, Triazophos und Trichlorphon;
Carbamate wie Aldicarb, Bendiocarb, α-2-(1-Methylpropyl)-phenylmethylcarbamat, Butocarboxim, Butoxycarboxim, Carbaryl, Carbofuran, Carbosulfan, Cloethocarb, Isoprocarb, Methomyl, Oxamyl, Pirimicarb, Promecarb, Propoxur und Thiodicarb;
Organosiliciumverbindungen, vorzugsweise Dimethyl(phenyl)silyl-methyl-3-phenoxybenzylether wie Dimethyl-(4-ethoxyphenyl)-silylmethyl-3-phenoxybenzylether oder
(Dimethylphenyl)-silyl-methyl-2-phenoxy-6-pyridylmethylether wie z.B. Dimethyl-(9-ethoxy-phenyl)-silylmethyl-2-phenoxy-6-pyridylmethylether oder [(Phenyl)-3-(3-phenoxyphenyl)-propyl](dimethyl)-silane wie z.B. (4-Ethoxyphenyl)-[3-(4-fluoro-3-phenoxyphenyl-propyl]dimethyl-silan, Silafluofen;
Pyrethroide wie Allethrin, Alphamethrin, Bioresmethrin, Byfenthrin, Cycloprothrin, Cyfluthrin, Decamethrin, Cyhalothrin, Cypermethrin, Deltamethrin, Alpha-cyano-3-phenyl-2-methylbenzyl-2,2-dimethyl-3-(2-chlor-2-trifluor-methylvinyl)cyclopropancarboxylat, Fenpropathrin, Fenfluthrin, Fenvalerate, Flucythrinate, Flumethrin, Fluvalinate, Permethrin, Resmethrin und Tralomethrin;
Nitroimine und Nitromethylene wie 1-[(6-Chlor-3-pyridinyl)-methyl]-4,5-dihydro-N-nitro-1H-imidazol-2-amin (Imidacloprid), N-[(6-Chlor-3-pyridyl)methyl-]N²-cyano-N¹-methylacetamide (NI-25);
Abamectin, AC 303, 630, Acephate, Acrinathrin, Alanycarb, Aldoxycarb, Aldrin, Amitraz, Azamethiphos, Bacillus thuringiensis, Phosmet, Phosphamidon, Phosphine, Prallethrin, Propaphos, Propetamphos, Prothoate, Pyraclofos, Pyrethrins, Pyridaben, Pyridafenthion, Pyriproxyfen, Quinalphos, RH-7988, Rotenone, Sodium fluoride, Sodium hexafluorosilicate, Sulfotep, Sulfuryl fluoride, Tar Oils, Teflubenzuron, Tefluthrin, Temephos, Terbufos, Tetrachlorvinphos, Tetramethrin, O-2-tert.-Butyl-pyrimidin-5-yl-o-isopropyl-phosphorothiate, Thiocyclam, Thiofanox, Thiometon, Tralomethrin, Triflumuron, Trimethacarb, Vamidothion, Verticillium Lacanii, XMC, Xylylcarb, Benfuracarb, Bensultap, Bifenthrin, Bioallethrin, MERbioallethrin (S)-cyclopentenyl isomer, Bromophos, Bromophos-ethyl, Buprofezin, Cadusafos, Calcium Polysulfide, Carbophenothion, Cartap, Chinomethionat, Chlordane, Chlorfenvinphos, Chlorfluazuron, Chlormephos, Chloropicrin, Chlorpyrifos, Cyanophos, Beta-Cyfluthrin, Alpha-cypermethrin, Cyophenothrin, Cyromazine, Dazomet, DDT, Demeton-S-methylsulphon, Diafenthiuron, Dialifos, Dicrotophos, Diflubenzuron, Dinoseb, Deoxabenzofos, Diaxacarb, Disulfoton, DNOC, Empenthrin, Endosulfan, EPN, Esfenvalerate, Ethiofencarb, Ethion, Etofenprox, Fenobucarb, Fenoxycarb, Fensulfothion, Fipronil, Flucycloxuron, Flufenprox, Flufenoxuron, Fonofos, Formetanate, Formothion, Fosmethilan, Furathiocarb, Heptachlor, Hexaflumuron, Hydramethylnon, Hydrogen Cyanide, Hydroprene, IPSP, Isazofos, Isofenphos, Isoprothiolane, Isoxathion, Iodfenphos, Kadethrin, Lindane, Malathion, Mecarbam, Mephosfolan, Mercurous, chloride, Metam, Metarthizium, anisopliae, Methacrifos, Methamidophos, Methidathion, Methiocarb, Methoprene, Methoxychlor, Methyl isothiocyanate, Metholcarb, Mevinphos, Monocrotophos, Naled, Neodiprion sertifer NPV, Nicotine, Omethoate, Oxydemeton-methyl, Pentachlorophenol, Petroleum oils, Phenothrin, Phenthoate, Phorate;

### Molluscicide:

Fentinacetate, Metaldehyde, Methiocarb. Niclosamide, Thiodicarb, Trimethacarb.

### Algicide:

Coppersulfate, Dichlororphen, Endothal, Fentinacetate, Quinodamine.

### Herbicide:

acetochlor, acifluorfen, aclonifen, acrolein, alachlor, alloxydim, ametryn, amidosulfuron, amitrole, ammonium sulfamate, anilofos, asulam atrazine, aziptrotryne, benazolin, benfluralin, benfuresate, bensulfuron, bensulfide, bentazone, benzofencap, benzthiazuron, bifenox, bilanafos, borax, dichlorprop, dichlorprop-P, diclofop, diethatyl, difenoxuron, difenzoquat, diflufenican, dimefuron, dimepiperate, dimethachlor, dimethametryn, dimethipin, dimethylarsinic acid, dinitramine, dinoseb, dinoseb, dinoseb acetate, dinoseb, bromacil, bromobutide, bromofenoxim, bromoxynil, butachlor, butamifos, fuenachlor, butralin, butylate, carbetamide, CGA 184927, chlormethoxyfen, chloramben, chlorbromuron, chlorbutam, chlorfurenol, chloridazon, chlorimuron, chlomitrofen, chloroacetic acid, achloropicrin, chlorotoluron, chloroxuron, chlorprepham, chlorsulfuron, chlorthal, chlorthiamid, cinmethylin, cinofulsuron, clethodim, clomazone, clomeprop, clopyralid, cyanamide, cyanazine, dinoseb acetate, dinoterb, diphenamid, dipropetryn, diquat, dithiopyr, diduron, DNOC, PPX-A 788, DPX-E96361, DSMA, eglinazine, endothal, EPTC, esprocarb, ethalfluralin, ethidimuron, ethofumesate, fenoxaprop, fenoxaprop-P, fenuron, flamprop, flamprop-M, flazasulfuron, fluazifop, fluazifop-P, fluchloralin, flumeturon, fluorocgycofen, fluoronitrofen, flupropanate, flurenol, fluridone, flurochloridone, fluoroxypyr, cycloate, cycloxydim, 2,4-D, daimuron, dalapon, dazomet, 2,4-DB, desmedipham, desmetryn, dicamba, dichlorbenil, isoproturon, isouron, isoxaben, isoxapyrifop, lactofen, lenacil, linuron, LS830556, MCPA, MCPA-thioethyl, MCPB, mecoprop, mecoprop-P, mefenacet, mefluidide, metam, metamitron, metazachlor, methabenzthiazuron, methazole, methoproptryne, methyldymron, methylisothiocyanate, metobromuron, fomosafen, fosamine, furyloxyfen, glufosinate, glyphosate, haloxyfop, hexazinone, imazamethabenz, imazapyr, imazaquin, imazethapyr, ioxynil, isopropalin, propyzamide, prosulfocab, pyrazolynate, pyrazolsulfuron, pyrazoxyfen, pyributicarb, pyridate, quinclorac, quinmerac, quinocloamine, quizalofop, quzizalofop-P, S-23121, sethoxydim, sifuron, simazine, simetryn, SMY 1500, sodium chlorate, sulfometuron, tar oils, TCA, metolachlor, metoxuron, metribzin, metsulfuron, molinate, monalide, monolinuron, MSMA, naproanilide, napropamide, naptalam, neburon, nicosulfuron, nipyraclofen, norflurazon, orbencarb, oaryzalin, oxadiazon, oxyfluorfen, paraquat, pebulate, pendimethalin, pentachlorophenol, pentaochlor, petroleum oils, phenmedipham, picloram, piperophos, pretilachlor, primisulfuron, prodiamine, proglinazine, propmeton, prometryn, propachlor, tebutam, tebuthiuron, terbacil, terbumeton, terbuthylazine, terbutryn, thiazafluoron, thifensulfuron, thiobencarb, thiocarbazil, tioclorim, tralkoxydim, tri-allate, triasulfuron, tribenzuron, triclopyr, tridiphane, trietazine, trifluralin, IBI-C4874 vernolate, propanil, propaquizafop, propazine, propham.

Die Gewichtsverhältnisse der Wirkstoffe in diesen Wirkstoffkombinationen können in relativ großen Bereichen variiert werden.

Vorzugsweise erhalten die Wirkstoffkombinationen den Wirkstoff zu 0,1 bis 99,9 %, insbesondere zu 1 bis 75 %, besonders bevorzugt 5 bis 50 %, wobei der Rest zu 100 % durch einen oder mehrere der obengenannten Mischungspartner ausgefüllt wird.

Die zum Schutz der technischen Materialien verwendeten mikrobiziden Mittel oder Konzentrate enthalten den Wirkstoff bzw. die Wirkstoffkombination in einer Konzentration von 0,01 und 95 Gew.-%, insbesondere 0,1 bis 60 Gew.-%.

Die Anwendungskonzentrationen der zu verwendenden Wirkstoffe bzw. der Wirkstoffkombinationen richtet sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Die erfindungsgemäßen Wirkstoffe bzw. Mittel ermöglichen in vorteilhafter Weise, die bisher verfügbaren mikrobiziden Mittel durch effektivere und weniger toxische zu ersetzen. Sie zeigen eine gute Stabilität und haben in vorteilhafter Weise ein breites Wirkungsspektrum.

Die nachfolgenden Beispiele dienen zur Verdeutlichung der Erfindung. Die Erfindung ist nicht auf die Beispiele beschränkt.

### Herstellungsbeispiele

### Beispiel 2

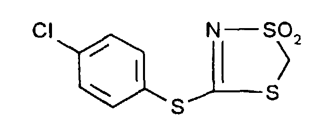

5 g (26 mmol) 1,4,2-Dithiazol-3-thiol-1,1-dioxid-K-Salz werden in 13 ml 20 %iger KOH gelöst und mit 1,82 g Cu-Pulver versetzt und auf ca. 0°C bis 5°C abgekühlt. Man tropft innerhalb von 30 Minuten die Diazoniumsalzlösung (I) zu, läßt auf Raumtemperatur kommen und saugt ab. Der Mutterkuchen wird mit Wasser gewaschen und anschließend in Essigsäureethylester aufgenommen. Man filtriert erneut und wäscht das Filtrat 2 x mit 2 N-HCl. Nach Trocknen über Na₂SO₄ wird eingedampft und an Kieselgel (Toluol/Essigsäureethylester = 10:1) chromatografiert.

Zur Entfernung farbiger Verunreinigungen wird mit wenig Diisopropylether verrührt und abgesaugt.

Ausbeute: 2,9 g ( 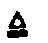 39 % der Theorie) eines leicht grauen Pulvers. Fp. = 172°C.

### Diazoniumsalzlösung (I):

3,31 g (26 mmol) 4-Chloranilin werden in 46 ml Wasser und 6,5 ml konz. HCl bei 2°C vorgelegt und mit einer Lösung von 1,9 g NaNO₂ in 15,6 ml H₂O tropfenweise versetzt und 1 Stunde nachgerührt.

Analog zu diesem Beispiel und entsprechend den obigen allgemeinen Angaben werden auch die in der nachstehenden Tabelle 1 aufgeführten Verbindungen der Formel (I) hergestellt.

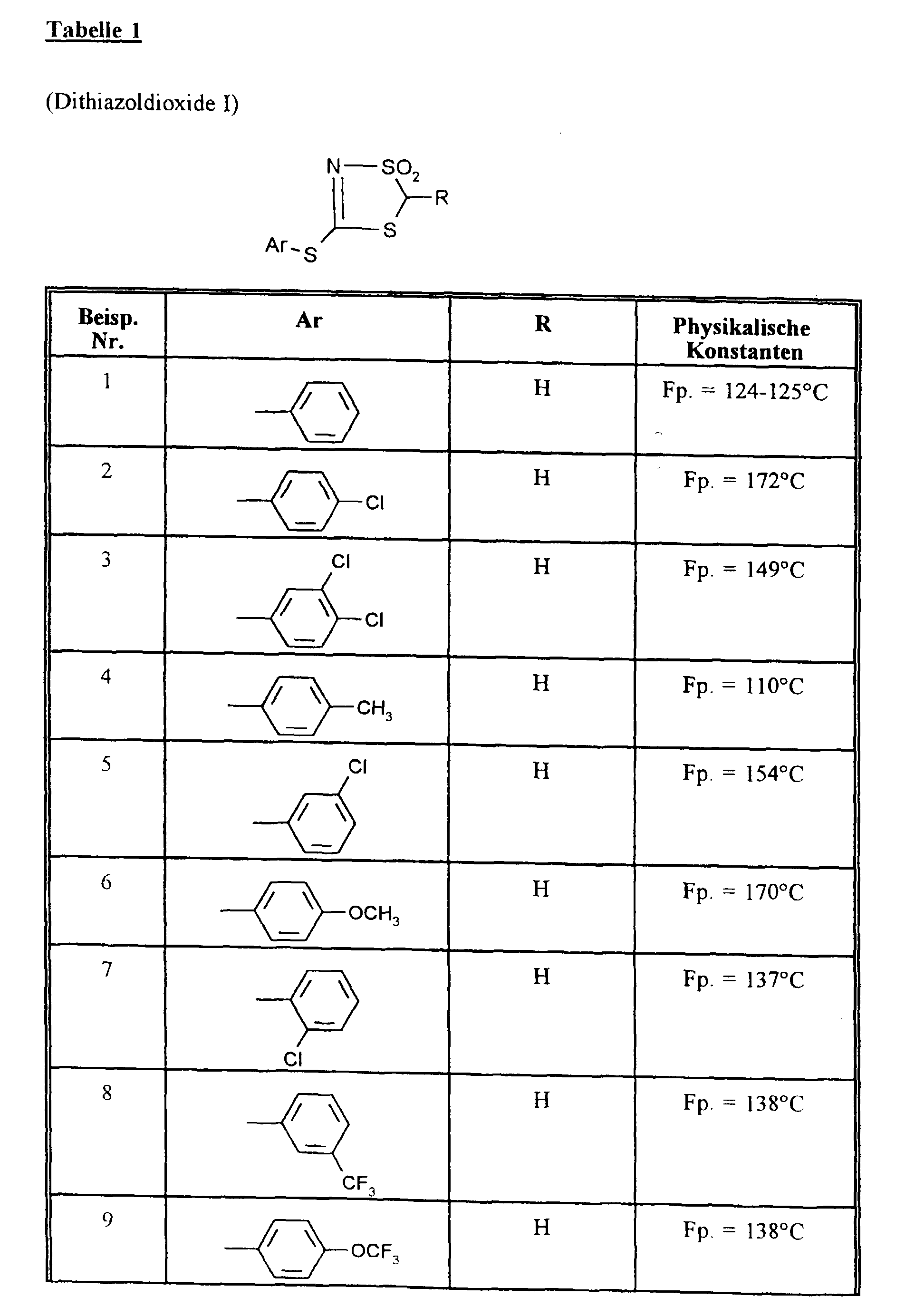

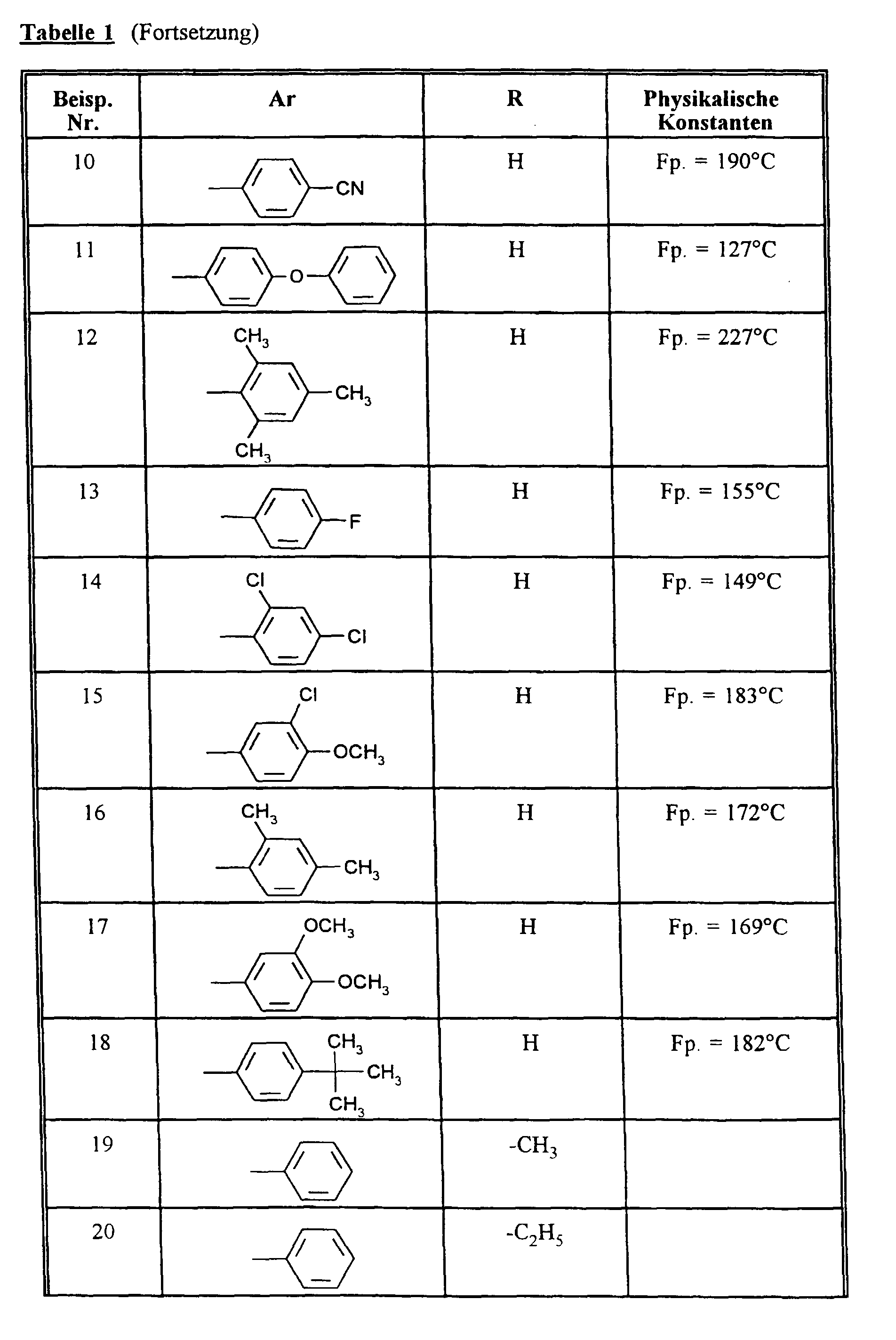

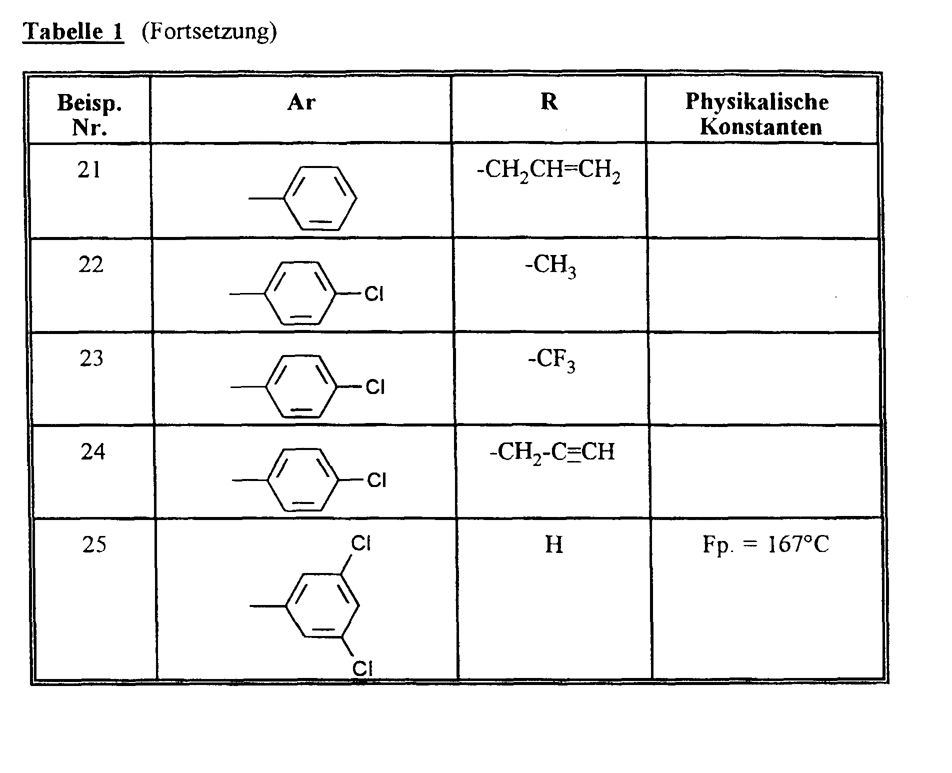

### Anwendungsbeispiel A

Zum Nachweis der Wirksamkeit gegen Pilze werden die minimalen HemmKonzentrationen (MHK) von erfindungsgemäßen Mitteln bestimmt:

Ein Agar, der unter Verwendung von Malzextrakt hergestellt wird, wird mit erfindungsgemäßen Wirkstoffen in Konzentrationen von 0,1 mg/l bis 5 000 mg/l versetzt. Nach Erstarren des Agars erfolgt Kontamination mit Reinkulturen der in der Tabelle 2 aufgeführten Testorganismen. Nach 2-wöchiger Lagerung bei 28°C und 60 bis 70 % relativer Luftfeuchtigkeit wird die MHK bestimmt. MHK ist die niedrigste Konzentration an Wirkstoff, bei der keinerlei Bewuchs durch die verwendete Mikrobenart erfolgt, sie ist in der nachstehenden Tabelle 2 angegeben.

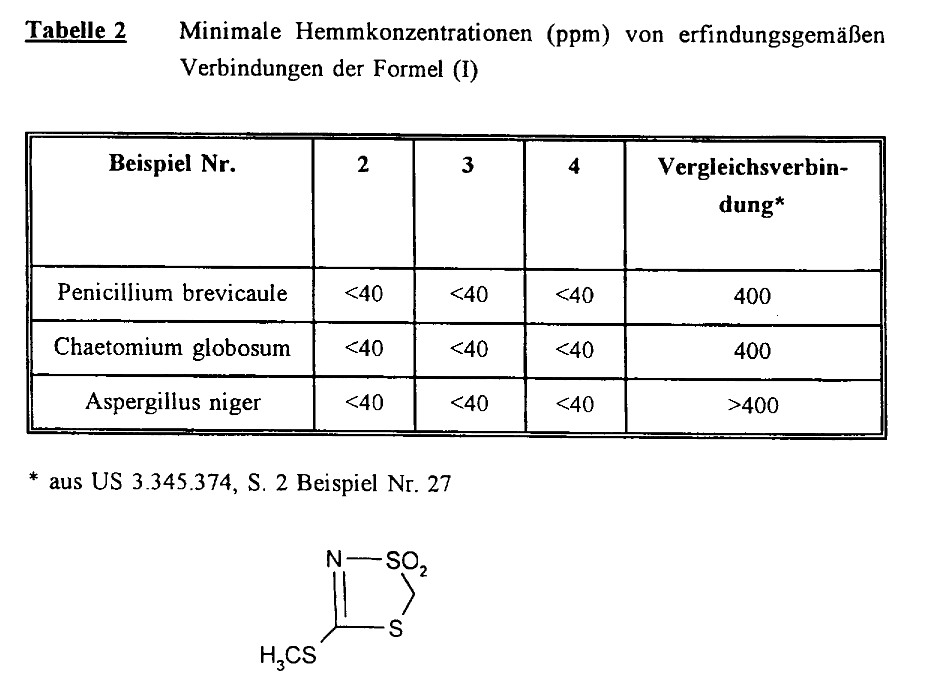

### Beispiel B

### Phytophthora-Test (Tomate) / protektiv

| | |
|---|---|
| Lösungsmittel | 4,7 Gewichtsteile Aceton |
| Emulgator | 0,3 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung taufeucht besprüht. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20°C aufgestellt.

3 Tage nach der Inokulation erfolgt die Auswertung.

Die Verbindung gemäß Herstellungsbeispiel 5 zeigt bei einer Wirkstoffkonzentration von 100 ppm einen Wirkungsgrad von 90 %.

### Beispiel C

### Plasmopara-Test (Reben) / protektiv

| | |
|---|---|
| Lösungsmittel | 4,7 Gewichtsteile Aceton |
| Emulgator | 0,3 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung taufeucht besprüht. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22°C und 100 % relativer Luftfeuchtigkeit. Anschließend werden die Pflanzen 5 Tage im Gewächshaus bei 21°C und ca. 90 % Luftfeuchtigkeit aufgestellt. Die Pflanzen werden dann angefeuchtet und 1 Tag in eine Feuchtkammer gestellt.

6 Tage nach der Inokulation erfolgt die Auswertung.

Die Verbindungen gemäß Herstellungsbeispiele 1, 5 und 6 zeigen bei einer Wirkstoffkonzentration von 100 ppm einen Wirkungsgrad von über 73 %.

### Beispiel D

### Botrytis-Test (Bohne) / protektiv

| | |
|---|---|
| Lösungsmittel | 4,7 Gewichtsteile Aceton |
| Emulgator | 0,3 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung taufeucht besprüht. Nach Antrocknen des Spritzbelages werden auf jedes Blatt 2 kleine mit Botrytis cinerea bewachsene Agarstücke aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Die Verbindungen gemäß der Herstellungsbeispiele 1, 2, 5, 6 und 14 zeigen bei einer Wirkstoffkonzentration von 500 ppm einen Wirkungsgrad von über 80 %.

## Patentansprüche

1. Verbindungen der allgemeinen Formel (I) 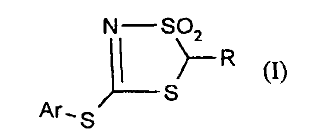 in welcher
R für Wasserstoff oder gegebenenfalls substituiertes Alkyl, Alkenyl oder Alkinyl steht und
Ar für gegebenenfalls substituiertes Aryl steht.

2. Verbindungen der Formel (I) gemäß Anspruch 1, in welcher
R für Wasserstoff, geradkettiges und verzweigtes Alkyl mit 1 ist 10 Kohlenstoffatomen, geradkettiges oder verzweigtes Alkenyl mit 2 bis 10 Kohlenstoffatomen oder geradkettiges oder verzweigtes Alkinyl mit 2 bis 10 Kohlenstoffatomen, welches gegebenenfalls ein- bis mehrfach, gleich oder verschieden substituiert ist durch Halogen, Alkoxy mit 1 bis 6 Kohlenstoffatomen, Halogenalkoxy mit 1 bis 6 Kohlenstoffatomen und 1 bis 9 gleichen oder verschiedenen Halogenatomen, Alkylthio mit 1 bis 6 Kohlenstoffatomen, Halogenalkylthio mit 1 bis 6 Kohlenstoffatomen und 1 bis 9 gleichen oder verschiedenen Halogenatomen, Acyl mit 1 bis 6 Kohlenstoffatomen, Acyloxy mit 1 bis 6 Kohlenstoffatomen, (Alkoxy)-carbonyl mit 1 bis 6 Kohlenstoffatomen, Amino, welches gegebenenfalls gleich oder verschieden substituiert ist durch Alkyl oder Aryl, gegebenenfalls substituiertes Phenoxy, Aryl, Pyridyl oder Pyridyloxy, Nitro oder Cyano, steht
und
Ar für Aryl steht,
welches gegebenenfalls ein- bis fünffach substituiert ist durch Halogen, Alkyl mit 1 bis 10 Kohlenstoffatomen, Halogenalkyl mit 1 bis 8 Kohlenstoffatomen und 1 bis 8 gleichen oder verschiedenen Halogenatomen, Alkoxy mit 1 bis 10 Kohlenstoffatomen, Halogenalkoxy mit 1 bis 8 Kohlenstoffatomen und 1 bis 8 gleichen oder verschiedenen Halogenatomen, Alkylthio mit 1 bis 10 Kohlenstoffatomen, Halogenalkylthio mit 1 bis 8 Kohlenstoffatomen und 1 bis 8 gleichen oder verschiedenen Halogenatomen, Amino, Monoalkylamino mit geradkettigen oder verzweigten Alkylresten mit 1 bis 6 Kohlenstoffatomen, dialkylamino mit gleichen oder verschiedenen, geradkettigen oder verzweigten Alkylresten mit je 1 bis 6 Kohlenstoffatomen, Cycloalkyl mit 1 bis 6 Kohlenstoffatomen, Methylendioxy, Difluormethylendioxy, Chlorfluormethylendioxy, Dichlormethylendioxy, Nitro oder Cyano.

3. Verbindungen der Formel (I) gemäß Anspruch 1, in welcher
R für Wasserstoff, geradkettiges oder verzweigtes Alkyl mit 1 bis 8 Kohlenstoffatomen, geradkettiges oder verzweigtes Alkenyl mit 2 bis 8 Kohlenstoffatomen oder gereadkettiges oder verzweigtes Alkinyl mit 2 bis 8 Kohlenstoffatomen, welches gegebenfalls einbis vierfach, gleich oder verschieden substituiert ist durch Fluor, Chlor, Alkoxy mit 1 bis 5 Kohlenstoffatomen, Halogenalkoxy mit 1 bis 5 Kohlenstoffatomen und 1 bis 5 Fluor- und/oder Chloratomen, Alylthio mit 1 bis 5 Kohlenstoffatomen, Halogenalkylthio mit 1 bis 5 Kohlenstoffatomen und 1 bis 5 fluor- und/oder Chloratomen, Acyl mit 1 bis 5 Kohlenstoffatomen, Acyloxy mit 1 bis 5 Kohlenstoffatomen, Alkoxycarbonyl mit 1 bis 5 Kohlenstoffatomen, Amino, welches gegebenenfalls gleich oder verschieden substituiert ist durch Alkyl mit 1 bis 4 Kohlenstoffatomen und/oder Phenyl, gegebenenfalls substituiertes Phenoxy, Aryl, Pyridyl, Pyridyloxy, Nitro oder Cyano, steht
und
Ar für Phenyl steht, welches gegebenenfalls ein- bis vierfach substituiert ist durch Fluor, Chlor, Alkyl mit 1 bis 8 Kohlenstoffatomen, Halogenalkyl mit 1 bis 6 Kohlenstoffatomen und 1 bis 6 Fluor- und/oder Chloratomen, Alkoxy mit 1 bis 8 Kohlenstoffatomen, Halogenalkoxy mit 1 bis 6 Kohlenstoffatomen und 1 bis 6 Fluor- und/oder Chloratomen, Alkylthio mit 1 bis 8 Kohlenstoffatomen, Halogenalkylthio mit 1 bis 6 Kohlenstoffatomen und 1 bis 6 Fluor- und/oder Chloratomen, Amino, Monoalkylamino mit Alkylresten von 1 bis 4 Kohlenstoffatomen, Dialkylamino mit gleichen oder verschiedenen Alkylresten mit jeweils 1 bis 4 Kohlenstoffatomen, Cycloalkyl mit 1 bis 6 Kohlenstoffatomen, Methylendioxy, Difluormethylendioxy, Chlorfluormethylendioxy, Dichlormethylendioxy, Nitro oder Cyano.

4. Verbindungen der Formel (I) gemäß Anspruch 1, in welcher
R für Wasserstoff, Methyl, Ethyl, n- und i-Propyl, n-, s-, i- und t-Butyl, Allyl und Propargyl steht, welche gegebenenfalls substituiert sind durch Fluor und/oder Chlor, Methoxy oder Methylthio, und Ar für Phenyl steht, welches gegebenenfalls ein- bis dreifach durch Chlor, Fluor, Methyl, Ethyl, n- und i-Propyl, n-, s-, i- und t-Butyl, Methoxy, Ethoxy, Trifluormethyl, Trifluormethoxy, Cyano und/oder Phenoxy substituiert ist.

5. Mikrobizide Mittel, gekennzeichnet durch einen Gehalt an mindestens einer Verbindung der Formel (I) nach Anspruch 1.

6. Verfahren zur Bekämpfung von Mikroorganismen, dadurch gekennzeichnet, daß man Verbindungen der Formel (I) nach Anspruch 1 auf Mikroorganismen und/oder ihren Lebensraum einwirken läßt.

7. Verfahren zur Herstellung von Verbindungen der Formel (I) nach Anspruch 1, dadurch gekennzeichnet, daß man die Salze der allgemeinen Formel (II) 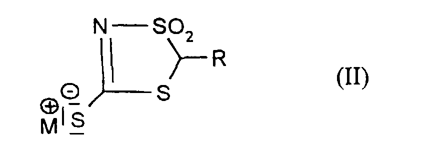 in welcher
R die oben angegebene Bedeutung hat und
M^{⊕} für ein Alkali- oder Erdalkaliion, im besonderen Na⁺, K⁺ steht,
mit Diazoniumsalzen der allgemeinen Formel (III)
Ar - N ≡ N^{⊕}A^{⊖} (III)
in welcher
Ar die oben angegebene Bedeutung hat und
A^{⊖} für das Anion einer Mineralsäure steht, in wäßrig/alkalischer Lösung, gegebenenfalls in Gegenwart eines Katalysators umsetzt.

8. Verwendung von Verbindungen der Formel (I) nach den Ansprüchen 1 bis 4 zur Bekämpfung von Mikroorganismen.

9. Verfahren zur Herstellung von mikrobiziden Mitteln, dadurch gekennzeichnet, daß man Verbindungen der Formel (I) nach den Ansprüchen 1 bis 4 mit Streckmitteln und/oder oberflächenaktiven Mitteln vermischt.

10. Verwendung von Verbindungen der Formel (I) nach Anspruch 1 zum Schutz von technischen Materialien vor mikrobiellem Befall.

## Claims

1. Compounds of the general formula (I) 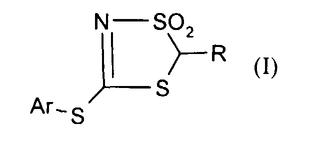 in which
R represents hydrogen or optionally substituted alkyl, alkenyl or alkinyl and
Ar represents optionally substituted aryl.

2. Compounds of the formula (I) according to Claim 1 in which
R represents hydrogen, straight-chain or branched alkyl having 1 to 10 carbon atoms, straight-chain or branched alkenyl having 2 to 10 carbon atoms or straight-chain or branched alkinyl having 2 to 10 carbon atoms, which is optionally mono- to polysubstituted by identical or different substituents from the group consisting of halogen, alkoxy having 1 to 6 carbon atoms, halogenoalkoxy having 1 to 6 carbon atoms and 1 to 9 identical or different halogen atoms, alkylthio having 1 to 6 carbon atoms, halogenoalkylthio having 1 to 6 carbon atoms and 1 to 9 identical or different halogen atoms, acyl having 1 to 6 carbon atoms, acyloxy having 1 to 6 carbon atoms, (alkoxy)-carbonyl having 1 to 6 carbon atoms, amino, which is optionally substituted by identical or different substituents from the group consisting of alkyl and aryl, optionally substituted phenoxy, aryl, pyridyl or pyridyloxy, nitro or cyano,
and
Ar represents aryl,
which is optionally mono- to pentasubstituted by halogen, alkyl having 1 to 10 carbon atoms, halogenoalkyl having 1 to 8 carbon atoms and 1 to 8 identical or different halogen atoms, alkoxy having 1 to 10 carbon atoms, halogenoalkoxy having 1 to 8 carbon atoms and 1 to 8 identical or different halogen atoms, alkylthio having 1 to 10 carbon atoms, halogenoalkylthio having 1 to 8 carbon atoms and 1 to 8 identical or different halogen atoms, amino, monoalkylamino having straight-chain or branched alkyl radicals having 1 to 6 carbon atoms, dialkylamino having identical or different, straight-chain or branched alkyl radicals having in each case 1 to 6 carbon atoms, cycloalkyl having 1 to 6 carbon atoms, methylenedioxy, difluoromethylenedioxy, chlorofluoromethylenedioxy, dichloromethylenedioxy, nitro or cyano.

3. Compounds of the formula (I) according to Claim 1 in which
R represents hydrogen, straight-chain or branched alkyl having 1 to 8 carbon atoms, straight-chain or branched alkenyl having 2 to 8 carbon atoms or straight-chain or branched alkinyl having 2 to 8 carbon atoms, which is optionally mono- to tetrasubstituted by identical or different substitutents from the group consisting of fluorine, chlorine, alkoxy having 1 to 5 carbon atoms, halogenoalkoxy having 1 to 5 carbon atoms and 1 to 5 fluorine and/or chlorine atoms, alkylthio having 1 to 5 carbon atoms, halogenoalkylthio having 1 to 5 carbon atoms and 1 to 5 fluorine and/or chlorine atoms, acyl having 1 to 5 carbon atoms, acyloxy having 1 to 5 carbon atoms, alkoxycarbonyl having 1 to 5 carbon atoms, amino which is optionally substituted by identical or different substituents from the group consisting of alkyl having 1 to 4 carbon atoms and phenyl, optionally substituted phenoxy, aryl, pyridyl, pyridyloxy, nitro or cyano,
and
Ar represents phenyl which is optionally mono- to tetrasubstituted by fluorine, chlorine, alkyl having 1 to 8 carbon atoms, halogenoalkyl having 1 to 6 carbon atoms and 1 to 6 fluorine and/or chlorine atoms, alkoxy having 1 to 8 carbon atoms, halogenoalkoxy having 1 to 6 carbon atoms and 1 to 6 fluorine and/or chlorine atoms, alkylthio having 1 to 8 carbon atoms, halogenoalkylthio having 1 to 6 carbon atoms and 1 to 6 fluorine and/or chlorine atoms, amino, monoalkylamino having alkyl radicals of 1 to 4 carbon atoms, dialkylamino having identical or different alkyl radicals having in each case 1 to 4 carbon atoms, cycloalkyl having 1 to 6 carbon atoms, methylenedioxy, difluoromethylenedioxy, chlorofluoromethylenedioxy, dichloromethylenedioxy, nitro or cyano.

4. Compounds of the formula (I) according to Claim 1, in which
R represents hydrogen, methyl, ethyl, n- or i-propyl, n-, s-, i- and t-butyl, allyl and propargyl which are optionally substituted by fluorine and/or chlorine, methoxy or methylthio, and Ar represents phenyl which is optionally mono- to trisubstituted by chlorine, fluorine, methyl, ethyl, n-and i-propyl, n-, s-, i- and t-butyl, methoxy, ethoxy, trifluoromethyl, trifluoromethoxy, cyano and/or phenoxy.

5. Microbicidal compositions, characterized in that they comprise at least one compound of the formula (I) according to Claim 1.

6. Method for controlling microorganisms, characterized in that compounds of the formula (I) according to Claim 1 are allowed to act on microorganisms and/or their habitat.

7. Process for preparing compounds of the formula (I) according to Claim 1, characterized in that the salts of the general formula (II) 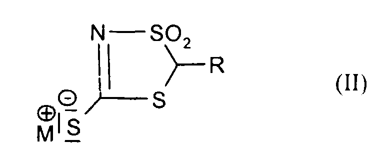 in which
R is as defined above and
M^{⊕} represents an alkali metal or alkaline earth metal ion, in particular Na⁺, K⁺
are reacted with diazonium salts of the general formula (III)
Ar - N ≡ N^{⊕}A^{⊖} (III)
in which
Ar is as defined above and
A^{⊖} represents the anion of a mineral acid, in aqueous/alkaline solution, if appropriate in the presence of a catalyst.

8. Use of compounds of the formula (I) according to Claims 1 to 4 for controlling microorganisms.

9. Process for preparing microbicidal compositions, characterized in that compounds of the formula (I) according to Claims 1 to 4 are mixed with extenders and/or surfactants.

10. Use of compounds of the formula (I) according to Claim 1 for protecting industrial materials against microbial attack.

## Revendications

1. Composés de formule générale (I) 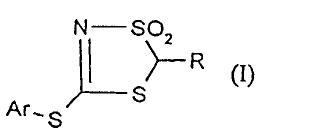 dans laquelle
R représente l'hydrogène ou un groupe alkyle, alcényle ou alcynyle éventuellement substitué et
Ar représente un groupe aryle éventuellement substitué.

2. Composés de formule (I) suivant la revendication 1, dans laquelle
R est l'hydrogène, un groupe alkyle linéaire ou ramifié ayant 1 à 10 atomes de carbone, un groupe alcényle linéaire ou ramifié ayant 2 à 10 atomes de carbone ou un groupe alcynyle linéaire ou ramifié ayant 2 à 10 atomes de carbone, qui est substitué, le cas échéant, une à plusieurs fois identiques ou différentes par un halogène, un groupe alkoxy ayant 1 à 6 atomes de carbone, halogénalkoxy ayant 1 à 6 atomes de carbone et 1 à 9 atomes d'halogènes identiques ou différents, alkylthio ayant 1 à 6 atomes de carbone, halogénalkylthio ayant 1 à 6 atomes de carbone et 1 à 9 atomes d'halogènes identiques ou différents, acyle ayant 1 à 6 atomes de carbone, acyloxy ayant 1 à 6 atomes de carbone, (alkoxy en C₁ à C₆)-carbonyle, amino, qui porte éventuellement des substituants alkyle ou aryle identiques ou différents, un groupe éventuellement substitué phénoxy, aryle, pyridyle ou pyridyloxy, un groupe nitro ou cyano,
et
Ar représente un groupe aryle,
qui est substitué, le cas échéant, une à cinq fois par un halogène, un radical alkyle ayant 1 à 10 atomes de carbone, halogénalkyle ayant 1 à 8 atomes de carbone et 1 à 8 atomes d'halogènes identiques ou différents, alkoxy ayant 1 à 10 atomes de carbone, halogénalkoxy ayant 1 à 8 atomes de carbone et 1 à 8 atomes d'halogènes identiques ou différents, alkylthio ayant 1 à 10 atomes de carbone, halogénalkylthio ayant 1 à 8 atomes de carbone et 1 à 8 atomes d'halogènes identiques ou différents, amino, mono-alkylamino portant des restes alkyle linéaires ou ramifiés ayant 1 à 6 atomes de carbone, dialkylamino portant des restes alkyle linéaires ou ramifiés identiques ou différents ayant chacun 1 à 6 atomes de carbone, cycloalkyle ayant 1 à 6 atomes de carbone, méthylènedioxy, difluorométhylènedioxy, chlorofluorométhylènedioxy, dichlorométhylènedioxy, nitro ou cyano.

3. Composés de formule (I) suivant la revendication 1, dans laquelle
R représente l'hydrogène, un groupe alkyle linéaire ou ramifié ayant 1 à 8 atomes de carbone, alcényle linéaire ou ramifié ayant 2 à 8 atomes de carbone ou alcynyle linéaire ou ramifié ayant 2 à 8 atomes de carbone, qui est substitué, le cas échéant, une à quatre fois identiques ou différentes par du fluor, du chlore, un groupe alkoxy ayant 1 à 5 atomes de carbone, halogénalkoxy ayant 1 à 5 atomes de carbone et 1 à 5 atomes de fluor et/ou de chlore, un groupe alkylthio ayant 1 à 5 atomes de carbone, halogénalkylthio ayant 1 à 5 atomes de carbone et 1 à 5 atomes de fluor et/ou de chlore, acyle ayant 1 à 5 atomes de carbone, acyloxy ayant 1 à 5 atomes de carbone, (alkoxy en C₁ à C₅)-carbonyle, amino qui porte éventuellement des substituants identiques ou différents, alkyle ayant 1 à 4 atomes de carbone et/ou phényle, phénoxy éventuellement substitué, aryle, pyridyle, pyridyloxy, nitro ou cyano,
et
Ar est un groupe phényle qui est substitué, le cas échéant, une à quatre fois par du fluor, du chlore, un groupe alkyle ayant 1 à 8 atomes de carbone, halogénalkyle ayant 1 à 6 atomes de carbone et 1 à 6 atomes de fluor et/ou de chlore, alkoxy ayant 1 à 8 atomes de carbone, halogénalkoxy ayant 1 à 6 atomes de carbone et 1 à 6 atomes de fluor et/ou de chlore, alkylthio ayant 1 à 8 atomes de carbone, halogénalkylthio ayant 1 à 6 atomes de carbone et 1 à 6 atomes de fluor et/ou de chlore, amino, mono-alkylamino portant des restes alkyle ayant 1 à 4 atomes de carbone, dialkylamino portant des restes alkyle identiques ou différents ayant chacun 1 à 4 atomes de carbone, cycloalkyle ayant 1 à 6 atomes de carbone, méthylènedioxy, difluorométhylènedioxy, chlorofluorométhylènedioxy, dichlorométhylènedioxy, nitro ou cyano.

4. Composés de formule (I) suivant la revendication 1, dans laquelle
R représente l'hydrogène, des groupes méthyle, éthyle, n-propyle, isopropyle, n-butyle, sec.-butyle, isobutyle, tertio-butyle, allyle et propargyle qui sont substitués, le cas échéant, par du fluor et/ou du chlore, un groupe méthoxy ou méthylthio, et Ar est un groupe phényle qui est substitué, le cas échéant, une à trois fois par du chlore, du fluor, un groupe méthyle, éthyle, n-propyle, isopropyle, n-butyle, sec.-butyle, isobutyle, tertio-butyle, méthoxy, éthoxy, trifluorométhyle, trifluorométhoxy, cyano et/ou phénoxy.

5. Compositions microbicides, caractérisées par une teneur en au moins un composé de formule (I) suivant la revendication 1.

6. Procédé pour combattre des micro-organismes, caractérisé en ce qu'on fait agir des composés de formule (I) suivant la revendication 1 sur les micro-organismes et/ou sur leur milieu.

7. Procédé de production de composés de formule (I) suivant la revendication 1, caractérisé en ce qu'on fait réagir les sels de formule générale (II) 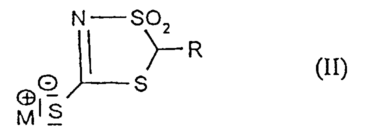 dans laquelle
R a la définition indiquée ci-dessus et
M^{⊕} représente un ion de métal alcalin ou alcalino-terreux, en particulier Na⁺, K⁺,
avec des sels de diazonium de formule générale (III)
Ar - N = N^{⊕} A^{⊖} (III)
dans laquelle
Ar a la définition indiquée ci-dessus et
A^{⊖} représente l'anion d'un acide minéral, en solution aqueuse/alcaline, éventuellement en présence d'un catalyseur.

8. Utilisation de composés de formule (I) suivant les revendications 1 à 4 pour combattre des micro-organismes.

9. Procédé de préparation de compositions microbicides, caractérisé en ce qu'on mélange des composés de formule (I) suivant les revendications 1 à 4 avec des diluants et/ou des agents tensio-actifs.

10. Utilisation de composés de formule (I) suivant la revendication 1 pour protéger d'une attaque microbienne des matériaux techniques.
